# EUROPEAN PATENT SPECIFICATION

(11) **EP 1 628 891 B1**
(45) Date of publication and mention of the grant of the patent: **16.05.2007**
(21) Application number: 04729127.3
(22) Date of filing: 23.04.2004
(51) Int. Cl.: B65D 79/02, G01N 33/02

(54) **METHOD FOR NOTICING CHANGES IN A PACKAGE, METHOD FOR LOCATING A PACKAGE, PACKAGE AND SYSTEM FOR LOCATING A PACKAGE**
VERFAHREN ZUR ERFASSUNG VON ÄNDERUNGEN IN EINER VERPACKUNG, VERFAHREN ZUR LOKALISIERUNG EINER VERPACKUNG, VERPACKUNG UND SYSTEM ZUR LOKALISIERUNG EINER VERPACKUNG
PROCEDE DE DETECTION DE CHANGEMENTS DANS UN EMBALLAGE, PROCEDE DE LOCALISATION D'UN EMBALLAGE, EMBALLAGE ET SYSTEME DE LOCALISATION D'UN EMBALLAGE

(30) Priority: 24.04.2003 FI 20030623
(43) Date of publication of application: 01.03.2006
(73) Proprietor: VALTION TEKNILLINEN TUTKIMUSKESKUS, 02150 Espoo 15 (FI)
(72) Inventor: HEILMANN, Jali, c/o Vtt Tietotekniikka, FI-02150 Espoo (FI); LINNA, Hannu, c/o Vtt Tietotekniikka, FI-02150 Espoo (FI); SIPILÄINEN-MALM, Thea, c/o Vtt Biotekniikka, FI-02150 Espoo (FI); HURME, Eero, c/o Vtt Biotekniikka, FI-02150 Espoo (FI); SMOLANDER, Maria, c/o Vtt Biotekniikka, FI-02150 Espoo (FI); NYKÄNEN, Heli, c/o Vtt Biotekniikka, FI-02510 Espoo (FI)
(74) Representative: Partio, Erja
(86) International application number: PCT/FI2004/000252
(87) International publication number: WO 2004/094262

(56) References cited:
- EP-A1- 0 627 363
- EP-A2- 0 117 390
- WO-A1-89/05761
- WO-A1-02/094672
- FI-B- 111 662

## Description

The invention relates to a method non-damaging the package for noticing a leakage, a change in the oxygen content and/or deterioration of the product in a package as defined in the preamble of claim 1, a method non-damaging the package for locating a package as defined in the preamble of claim 10, a package as defined in the preamble of claim 19, and a system as defined in the preamble of claim 23.

In the methods of the invention for noticing a leakage in a package, a change in the oxygen content, or deterioration of a product in the package, as well as for identifying the package, an indicator is used that changes its colour to indicate a change in the state of the package. Previously known is to use in packages various indicators that react e.g. as a result of oxygen passed into the package or as a result of a change in the pH, indicating the discolouration. For example, from publications US 4772707, JP 55-41875, JP 61-152299, JP 62-259059 and FI 94802 various indicators to be used in packages are known.

Publication US 4 349 509 discloses a foil-like oxygen indicator which is used e.g. in long-term vacuum packages of foodstuffs. The oxygen indicator colour mixture as disclosed in the publication is printed on a separate substrate to form the indicator using the aniline printing, gravure printing or letterpress technique. The indicator formed by the oxygen indicator colouring agent and substrate that indicates the presence of oxygen is separately placed into a storage package of a foodstuff.

Publication EP 627 363 discloses an oxygen and leakage indicator to be attached to a packaging material by printing.

Thus, an indicator colouring agent has been previously attached to a substrate to be placed in a package by means of various printing techniques such as the aniline printing, gravure printing or the letterpress technique. The aniline printing technique is a letterpress method operating by rotation technique in which the printing plate is flexible and the impression cylinder hard. The aforementioned printing methods can be used on the planar surfaces of rough, thin or flexible materials.

Publication EP 117 390 discloses an indicator that describes the effect of environment on the package, the indicator including a changing and a non-changing area as its reflecting power is concerned. The area that is changing in respect of its reflecting power can be printed e.g. using the inkjet technique. The publication does not disclose the printing of an indicator in conjunction with the packaging of the product.

The problem with the prior-art indicators is that it is not possible to manufacture and attach them in conjunction with the packaging of the product on a packaging line, instead the indicator is manufactured separately prior to the packaging phase. Further, the indicators must be stored prior to attaching them to the package as the prior-art printing techniques comprise complicated processes which cannot be used on the packaging line of the product. Further, the manufacturing, handling and specifically the storage of prior-art indicators in which the indicator colour mixture has been put on a separate substrate, such as e.g. an adhesive label or foil, is difficult as they tend to react with the factor that causes the change, e.g. with the oxygen of air.

Further, the prior-art indicators cannot simultaneously function as the identifiers of the package because the indicator itself has been manufactured prior to the packaging phase of the product. Thus, besides the indicator, it has been necessary to provide the package with a separate code for identifying and locating purposes, which adds to the handling phases of the packaging process.

The objective of the present invention is to overcome the aforementioned disadvantages. One specific objective of the invention is to disclose a novel and simple method for attaching the indicator directly to the packaging material in conjunction with the packaging on the packaging line. Further, the method enables the manufacturing of an individual, product-specific indicator on the packaging line. The objective of the dripping technique in accordance with the invention is to achieve a method which overcomes the problems of the prior-art indicators as the manufacturing, handling and specifically the storage are concerned. One further objective of the invention is to disclose a method for attaching the indicator to the packaging material in a manner enabling the use of the indicator as an identifier for individualising the package. The identifier included in the indicator enables the identification and locating of the packages included in the same batch, and if desired, also the sorting of the products based on this information.

As for the features characteristic of the invention, reference is made to them in the claims.

The invention is based on a research work carried out in which it was unexpectedly noticed that the use of the dripping technique is very well suitable for putting the indicator colour mixture in the packaging material in conjunction with the packaging.

The invention is based on a method in which the leakage, change in the oxygen content and/or deterioration of a product is shown without damaging the package by means of the discolouration of an indicator attached to it. According to the invention, the indicator is attached, using the dripping technique, directly to the surface of the packaging material just before sealing the package. In the dripping technique, the indicator is attached to the packaging material digitally using any printing technique based on dripping, e.g. using an inkjet printer or some other, e.g. an electrostatic technique. The indicator is preferably attached using the inkjet technique. The inkjet technique is based on a continuous piezo electric or thermal inkjet printing. A printing based on the dripping technique, such as e.g. the inkjet printing, is a contactless, additive method in which the desired amount of the material to be printed is transferred into the desired spots of the packaging material by computer control. In the method it is possible to print several different materials simultaneously from several nozzles.

The indicator to be attached to the surface of the packaging material is operative when printing, or is brought into working order after the printing.

The components of the indicator can be mixed before attaching the indicator to the surface of the packaging material, or alternatively, the components of the indicator can be attached to the surface of the packaging material by steps. After attaching to the packaging material, the indicator can be activated and/or its activating can be accelerated by momentarily subjecting it to conditions that cause and/or accelerate activation. The conditions can be reached e.g. by heat and/or light energy and/or by some other energy or technique suitable for the activation of an indicator.

The indicator attached to a packaging material can react to the oxygen from outside, indicating the ageing of the package. The indicator also reacts through leakage to the oxygen passed into the package. Further, the indicator can function as a deterioration indicator through an indirect mechanism. In that case, the compounds created in the deterioration of the product can cause the discolouration of the indicator through a suitably selected catalyst, e.g. an enzyme, or through the change in the pH. The susceptibility of the indicator can be so adjusted that no separate, active oxygen remover is needed inside the package. The indicator to be printed can be protected from direct contact with the packaged product using a separately printable protective layer, e.g. a lacquer layer.

The substance to be printed can be particle-based or be soluble. The substance to be printed can be e.g. water-based in which water is the main liquid component into which the colouring agent particles have been dispersed in the liquid. Alternatively, the substance can be based on thermoplastic, wax, or paraffin-based substances or combinations thereof, which have properties of both a soluble and a particle-based substance. In the invention, the substance to be printed comprises an indicator colour mixture, a binding agent and/or a solvent. The indicator can contain also other additives to be used in printing/colouring agents and indicators, such as a pH regulator, reducer, humidity maintainer, enzyme, substrate, plastifizer, wax, oxygen absorbent and/or commercial lacquers. As its composition is concerned, the indicator in accordance with the present invention need not be as viscous as the indicators used in the printing methods.

The composition of the indicator colour mixture is selected according to the product to be packed from indicator colours and colour mixtures generally known in the field. The colouring agents can be e.g. oxidation/reduction colours such as methylene blue, gallosyanine, reazurine and methylene red, pH colours and pH colours such as indigo carmine and bromo phenolic blue.

A binding agent is necessary when printing an indicator on a non-absorbing substrate such as a plastic surface. The binding agent can be selected according to the substrate to be printed and to suit the printer. The binding agent can be soluble or a dispersing polymer consisting of e.g. nitrocellulose, carboxy-methyl cellulose, polymer the starting materials of which can be e.g. butadiene, styrene, vinyl acetate, acrylic acid, methacrylic acid and/or esters thereof.

The reducing and pH regulating agents can include e.g. reducing sugars such as D(+) glucose, organic acids such as ascorbic acid and citric acid, and further sodium ascorbate, sodium sulphite, sodium bisulphite, sodium disulphite, sodium pyrophosphate, calcium ascorbate, dithionite, triethylamine, ammonium hydroxide, metallic powders such as iron and zinc, metallic salts such as several iron compounds. Humidity maintainers can include polyethylene glycol, glycerol, propylene glycol, sorbitol, erythritol. The solvent can include water and alcohols such as ethanol, methanol and isopropyl alcohol. The enzyme can be laccase, glucose oxidase and peroksidase. As mentioned above, the indicator can also include a substrate and/or an oxygen absorbent. When using enzymes, among substrates, a substrate typical of each enzyme comes into question. Various indicators to be used in packages are known e.g. from patent EP 936 999. Possible oxygen absorbents have been described e.g. in patent FI 94802.

The visible or optically measurable discolouration of an indicator happens as a result of the oxidation of the indicator colour caused by the growth of the oxygen content of the package and/or as a result of the change in the pH caused by the deterioration of the packaged product. In other words, the colouring agent is so prepared that it is operative, i.e. in a reduced and/or active state, when printing or directly after the printing, it remains reduced or unchanged in an undamaged package in which the packaged product is not spoilt, and when the package leaks or as a result of the opening of the package or as a result of the deterioration of the packaged product clearly changes its visible or optically readable colour

The indicator is prepared by conventional preparation/mixing techniques of colouring agents. The indicator can be prepared just before printing into the packaging material and packaging of the product. The indicator can also be prepared beforehand and stored either in a reduced or oxidised state. If the indicator is stored in an oxidised state, it can be reduced at the moment of packaging. The reducing agent can be e.g. printed into the material just before packaging the product. The reduction can also be implemented by adding to the indicator mixture a reducing compound and/or by subjecting the indicator after the packaging momentarily to conditions accelerating the reaction, e.g. to heat or light. In addition, it is possible to add between the layers and/or on top of them a protective layer preventing the oxidation and drying of the indicator.

In one embodiment, the indicator can be mixed at room temperature and after that be reduced in an accelerated manner by heating the solution (less than 100°C), after which the reduced indicator solution is stored as protected from oxygen, e.g. in sealed inkjet sets. Storing in a reduced state may require a separate active oxygen remover to be used in the package.

An indicator attached to a package may change its colour at the wavelength of visible light, 400-780 nm, or at the wavelengths of ultraviolet radiation, 100-400 nm. The discolouration of the indicator can be observed visually or by means of automatic reading techniques.

The packaging material can be pulp, plastic and/or glass-based and/or any other generally used packaging material. The pulp-based material can be e.g. a surface treated or untreated paper, cardboard, dissolving pulp-based foil material or other pulp-based or polyacetate-based material. The plastic material can be e.g. polyethylene, polypropylene, other polyolefin, polyester, polystyrene, polyamide or any other plastic material generally used as a packaging material. Further, the packaging material can consist of the laminate of the aforementioned or other known packaging materials or of some other kind of compound material. The packaging material can also be coated. Further, the packaging material can be corona treated or treated in some other manner to reduce the surface energy of the material.

Further, the invention is based on a method non-damaging the package for locating the package by attaching an indicator to the packaging material in conjunction with the packaging. The indicator is attached directly to the surface of the packaging material before sealing the package using the dripping technique, such as e.g. the inkjet technique. The locating of the package is performed by means of the indicator, which is made the identifier individualising the package. The identifier formed by the indicator can be any identifier generally used in the packaging field, such as a two- or three-dimensional identifier. The identifier can further be a compound bar identifier in which a linear, two- or three-dimensional bar identifier has been combined to form one symbol. The identifier can also be an HTML bar identifier. The identifier can comprise e.g. the batch number and manufacturing time of the product.

In one embodiment of the invention, the identifier formed by the indicator attached to the package using the dripping technique is read. The identifier can be read by any automatic reading technique, such as a laser scanner, a CCD scanner, or by utilising dot, line or matrix-structured detection heads, preferably CCD or CMOS cells. Next, the read information included in the identifier can be transmitted by connecting the detection head to a computer, or by using wireless data transfer technologies. Further, by means of the identifier, the product data of the package are retrieved from a database, by means of which it is possible to locate the packages included in the same batch with the package. Finally, a sorting decision is made, as the other packages are concerned, i.e. for example, with deteriorated foodstuffs, the packages included in the same batch are recalled.

Further, the invention is based on a package which is formed in a manner as presented above. The package of the invention can consist of pulp, plastic and/or glass-based and/or any other generally used packaging material and/or combinations of these, as presented above. The package can preferably be used in the packages of foodstuffs, pharmaceuticals or cosmetics. By means of the package of the invention it is possible to notice a leakage of the package, a change in the oxygen content and/or the deterioration of the product by means of an indicator attached to the package. Further, by means of an identifier formed by the indicator attached to the package it is possible to individualise the package and to identify it by means of this.

Further, the invention is based on a system for locating a package. The system comprises a dripping device for attaching the indicator to the packaging material, a reading and/or identifying device for reading and/or identifying the indicator attached to the package, and a database enabling the locating of the packages included in the same batch with the package.

In one embodiment of the invention, the dripping device is an inkjet printer. The reading and/or identifying device can be e.g. a laser scanner, a CCD scanner and/or a device utilising dot, line or matrix-structured detection heads, preferably CCD or CMOS cells.

The method of the invention has the advantage that by means of a method simpler than before, the attaching of an indicator to the packaging material can be performed such that besides indicating a leakage of the package, a change in the oxygen content and/or the deterioration of the product inside the package, the one and the same indicator can function as an identifier to individualise the package for identifying and locating the package. Thus, the package need not be separately marked with a code, which makes e.g. the processes of a packaging line simpler than before. The methods of the invention have the advantage that the preparing of indicators on separate substrates to be attached to packages is not necessary, instead the desired indicator colour mixture can be prepared as late as on the packaging line, just before attaching the indicator to the surface of the packaging material.

Further, the advantage of the printing technique of the invention compared to prior art, such as e.g. to impression techniques, is that the amount of the transferable material and its placing on the substrate is controlled e.g. by the aid of the computer. The technique has the advantage that it is more flexible than the methods in which the layering and embossing are performed using various impression surfaces or masks. For example, the material is transferred only the desired amount to the desired points. The technique in question consumes very little material. The digitality further enables e.g. fast manufacturing of prototypes of electronics systems and small series. As the printing is a contactless method, the printing head can be freely moved in relation to the printing platform. This enables printing also on non-planar surfaces. The method of the present invention enables an exact adjustment, but also the printing of big surfaces is advantageous and dependable. Also the formation of in situ layering and three-dimensional structures is possible by means of the methods of the present invention. Further, it is possible to print different materials simultaneously from different nozzles.

Further, the dripping method of the invention has the advantage that it is an environmentally friendly manufacturing method. No toxic solvents are used in the method, and the generation of waste is very low. The method in question is considerably more environmentally friendly than multi-phased evaporation, etching and coating methods. Further, the manufacturing technology of the invention, as well as the components used in it are very inexpensive in respect of costs.

In the following, the invention will be described in detail by means of embodiment examples with reference to the accompanying drawings, in which
Fig. 1 represents the attaching of an indicator to the surface of a packaging material disposed on the packaging line using the inkjet technique; and
Fig. 2 represents an identifier formed by the indicator attached to the surface of the packaging material.

### EXAMPLE 1

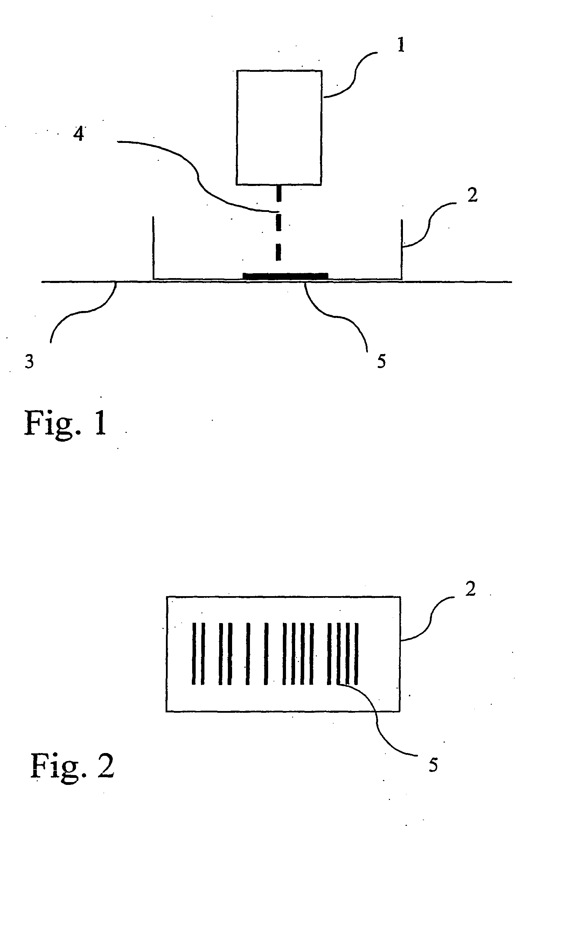

Fig. 1 shows a package 2 disposed on a packaging line 3, into which package a perishable foodstuff is packaged. The package consists of a packaging material that permeates little or not at all oxygen, and forms a mainly sealed packaging space.

Just before placing the foodstuff into the package and sealing the package, an indicator 4 is printed on the surface of the packaging material using an inkjet printer 1. At the same time, an identifier 5 is made out of the indicator 4, which is shown in Fig. 2. The identifier individualises the package. The identifier 5 formed by the indicator 4 is printed in this embodiment into the shape of a bar code including the batch number and manufacturing time of the foodstuff to be placed into it.

The indicator 4 to be attached to the surface of the packaging material 2 includes in this embodiment e.g. one or more colouring agents which react to a factor indicating the deterioration of a foodstuff and/or to a change in the oxygen content of the package, a binding agent and a solvent. The technique in accordance with the invention enables the adjustment of the indicator colour mixture in such a manner that it is not susceptible to oxygen, in which case no separate oxygen remover is needed in the package. Only a bigger amount of oxygen penetrating into the package causes the discolouration of the indicator, which is visible, without doing damage to the package, from outside the package, through a transparent place in the package.

In addition to the aforementioned facts, the indicator 4 comprises an oxidising enzyme which requires oxygen to operate, and a substrate of the enzyme. The enzyme and substrate are so selected that the oxidation product produced in the enzymatic reaction also is a typical volatile compound produced in the deterioration caused by microbiological deterioration. Thus, as the compound is generated inside the package 2 as the foodstuff goes bad, the compound causes in the indicator 4 a reaction which is observed as a discolouration of the indicator 4.

The indicator 4, its colouring agent as well as the enzyme and the substrate are in accordance with the prior-art technique, and are thus not described more fully herein.

As the package 2 ages, oxygen gradually penetrates into the package, and the foodstuff in the package 2 goes bad. Alternatively, the package 2 may unnoticeably be damaged e.g. during the transportation or placing on a shelf. The indicator 4 printed into the packaging material 2 reacts e.g. to the penetration of oxygen into the package 2, indicating by the discolouration the ageing of the package 2 in a certain time after the package 2 has been subjected to the environment.

By its discolouration, the indicator 4 effectively indicates the ageing of the package, the deterioration of the package and/or the breakage of the package. Based on the discolouration of the indicator 4, a consumer is able to directly see in the indicator the time the package 2 has been unshielded from the environmental effects, e.g. on a shelf of a shop, or the moment of packaging.

Once a discolouration has been noticed in the indicator 4 of a foodstuff package 2 in accordance with the example, the bar code 5 contained in the indicator 4 is read by means of an identifying device. Once the product's batch number and manufacturing time contained in the identifier 5 in question have been found out, the locations of the other packages included in the same batch are checked from the database. After this, the destinations in question are notified, and the situation with the corresponding products is checked. The deteriorated products are recalled.

### EXAMPLE 2

The objective of the test was to indicate the discolouration of a printed indicator colour by the action of oxygen. The following indicator colouring agent was prepared:
25 ml methylene blue/water-solution (10 mg methylene blue/1 ml water)
25 ml Na₂SO₃ (water-solution, 50 mg Na₂SO₃/ 1 ml water)
10 ml polyethylene glycol 400

The mixture was heated to be boiling and was cooled to room temperature. Lacquer (Sicpa 1100, pH 6.2) was mixed into the mixture in relation 1:1. The freshly prepared indicator colour mixture was put into the cartridge of an inkjet printer and printed using the inkjet printer onto a corona treated bright polyethylene foil. The printed colour was light right after the printing, but got blue by the action of oxygen in 2 hours.

### EXAMPLE 3

The objective of the test was to indicate the discolouration of a printed indicator colour by the action of organic acids (are produced as foodstuff deteriorate) in a confined space. The following indicator colouring agent was prepared:
25 ml reazurine/water-solution (2 mg/1 ml water
25 ml Sicpa 1100 lacquer

The freshly prepared indicator colouring agent was put into the cartridge of an inkjet printer and printed using the inkjet printer onto a corona treated bright polyethylene foil. The printed colour was bluish-violet right after the printing. 1 x 1 pieces of indicators printed on the polyethylene foil were sealed into glass flasks of 50 ml which contained 200 ppm formic acid, 200 ppm acetic acid or water. The indicators were not in contact with the acid or water. The colour of the indicators sealed in the flasks that contained acids turned red in about 2 hours. In the flasks that contained water the colour of the indicators remained the same.

The invention is not limited solely to the embodiment examples referred to above, instead various modifications are possible within the scope of the inventive idea defined by the claims.

## Claims

1. A method non-damaging the package for noticing a change caused by a leakage, the oxygen content and/or deterioration of a product in a package by attaching an indicator to the packaging material, **characterised in that**
- attaching an indicator using the dripping technique directly to the surface of the packaging material in conjunction with the packaging before sealing the package; and
- indicating by the discolouration of the indicator the leakage of the package, a change in the oxygen content and/or the deterioration of the product.

2. The method as defined in claim 1, **characterised in that** the indicator is attached to the surface of the packaging material using the inkjet technique.

3. The method as defined in claim 1 or 2, **characterised in that** the indicator is operative when printing or is brought into working order after the printing.

4. The method as defined in any one of claims 1-3, **characterised in that** the components of the indicator are mixed prior to attaching the indicator to the surface of the packaging material, or the components of the indicator are attached to the surface of the packaging material step by step.

5. The method as defined in any one of claims 1-4, **characterised in that** the indicator is activated by subjecting it to conditions that cause and/or accelerate activation.

6. The method as defined in any one of claims 1-5, **characterised in that** the indicator comprises a colouring agent mixture which reacts to a change in the oxygen content, to a change in the pH in the package, and/or to a compound produced in conjunction with the deterioration of the product.

7. The method as defined in any one of claims 1-6, **characterised in that** the colour of the indicator changes as a result of oxidation and/or through an indirect mechanism, which indirect mechanism is based on the pH change and/or on a reaction caused by a catalyst, preferably an enzyme.

8. The method as defined in any one of claims 1-7, **characterised in that** the discolouration of the indicator is noticed visually and/or is read by means of automatic reading techniques.

9. The method as defined in any one of claims 1-8, **characterised in that** the packaging material can be pulp, plastic and/or glass-based and/or any other generally used packaging material and/or a compound material of these.

10. A method non-damaging the package for locating the package by attaching an indicator to the packaging material, **characterised in that**
- attaching an indicator using the dripping technique directly to the surface of the packaging material in conjunction with the packaging before sealing the package; and
- locating the package by means of the indicator.

11. The method as defined in claim 10, **characterised in that** the indicator is attached to the surface of the packaging material using the inkjet technique.

12. The method as defined in claim 10 or 11, **characterised in that** an identifier individualising the package is formed from the indicator..

13. The method as defined in claim 12, **characterised in that** the identifier is a one-, two- or three-dimensional identifier; a compound bar identifier in which a linear, two- or three-dimensional bar identifier has been combined to form one symbol; or an HTML bar identifier.

14. The method as defined in claim 12 or 13, **characterised in that** the identifier comprises the batch number and manufacturing time of the product.

15. The method as defined in any one of claims 10-14, **characterised in that**
- reading the identifier formed by the indicator attached to the package,
- retrieving by means of the read identifier the production data of the package from the database,
- locating the packages included in the same batch.

16. The method as defined in claim 15, **characterised in that** a sorting decision is made as the located packages included in the same batch are concerned.

17. The method as defined in claim 15 or 16, **characterised in that** the identifier is read by means of automatic reading techniques.

18. The method as defined in any one of claims 15-17, **characterised in that** the read information included in the identifier is transmitted by connecting the detection head to a computer and/or by means of wireless data transfer technologies.

19. A package for noticing a change caused by a leakage, the oxygen content, or deterioration of a product in a package using an indicator attached to it, which package consists of pulp, plastic and/or glass-based and/or any other generally used packaging material and/or a compound material of these, **characterised in that** the package has been formed by attaching the indicator using the dripping technique to the surface of the packaging material in conjunction with the packaging before sealing the package.

20. The package as defined in claim 19, **characterised in that** the indicator has been attached to the surface of the packaging material using the inkjet technique.

21. The package as defined in claim 19 or 20, **characterised in that** the indicator has been formed to form an identifier which individualises the package.

22. The package as defined in any one of claims 19-21, **characterised in that** the package is a foodstuff, pharmaceutical or a cosmetics package.

23. A system for locating a package, **characterised in that** the system comprises:
- a dripping device for attaching the indicator to the package in conjunction with the packaging,
- a reading and/or identifying device for reading and/or identifying the indicator attached to the package,
- a database for locating the packages included in the same batch with the package.

24. The system as defined in claim 23, **characterised in that** the reading and/or identifying device comprises a laser scanner, a CCD scanner and/or dot, line or matrix-structured detection heads, preferably CCD or CMOS cells.

25. A system as defined in claim 23, **characterised in that** the reading and/or identifying device comprises a laser scanner, a CCD scanner and/or a device utilising dot, line or matrix-structured detection heads, preferably CCD or CMOS cells.

## Patentansprüche

1. Verfahren, bei dem die Verpackung nicht beschädigt wird, um eine durch eine Undichtigkeit verursachte Veränderung, den Sauerstoffgehalt und/oder Verschlechterung eines Erzeugnisses in einer Verpackung durch Anbringen einer Anzeigeeinrichtung an dem Verpackungsmaterial mitzuteilen,
**dadurch gekennzeichnet, dass**
eine Anzeigeeinrichtung unter Verwendung des Auftropfverfahrens direkt an der Oberfläche des Verpackungsmaterial in Verbindung mit dem Verpacken vor Abdichten der Verpackung angebracht wird; und
die Undichtigkeit der Verpackung, eine Veränderung des Sauerstoffgehaltes und/oder die Verschlechterung des Erzeugnisses durch die Entfärbung der Anzeigeeinrichtung angezeigt wird.

2. Verfahren nach Anspruch 1, **dadurch gekennzeichnet, dass** die Anzeigeeinrichtung unter Verwendung des Tintenstrahlverfahrens an der Oberfläche des Verpackungsmaterials angebracht wird.

3. Verfahren nach Anspruch 1 oder 2, **dadurch gekennzeichnet, dass** die Anzeigeeinrichtung funktioniert, wenn sie aufgedruckt wird oder nach Aufdrucken in Funktionszustand gebracht wird.

4. Verfahren nach einem der Ansprüche 1-3, **dadurch gekennzeichnet, dass** die Komponenten der Anzeigeeinrichtung vermischt werden, bevor die Anzeigeeinrichtung an der Oberfläche des Verpackungsmaterials angebracht wird, oder die Komponenten der Anzeigeeinrichtung Schritt für Schritt an der Oberfläche des Verpackungsmaterials angebracht werden.

5. Verfahren nach einem der Ansprüche 1-4, **dadurch gekennzeichnet, dass** die Anzeigeeinrichtung aktiviert wird, indem sie Bedingungen ausgesetzt wird, die Aktivierung verursachen und/oder beschleunigen.

6. Verfahren nach einem der Ansprüche 1-5, **dadurch gekennzeichnet, dass** die Anzeigeeinrichtung eine Färbemittelgemisch umfasst, das auf eine Veränderung des Sauerstoffgehaltes, auf eine Veränderung des pH-Wertes in der Verpackung und/oder auf eine Verbindung reagiert, die im Zusammenhang mit der Verschlechterung des Erzeugnisses erzeugt wird.

7. Verfahren nach einem der Ansprüche 1-6, **dadurch gekennzeichnet, dass** sich die Farbe der Anzeigeeinrichtung aufgrund von Oxidation und/oder eines indirekten Mechanismus ändert, wobei der indirekte Mechanismus auf der pH-Wert-Änderung und/oder einer Reaktion basiert, die durch einen Katalysator, vorzugsweise ein Enzym, verursacht wird.

8. Verfahren nach einem der Ansprüche 1-7, **dadurch gekennzeichnet, dass** die Entfärbung der Anzeigeeinrichtung visuell mitgeteilt wird und/oder mittels automatischer Leseverfahren gelesen wird.

9. Verfahren nach einem der Ansprüche 1-8, **dadurch gekennzeichnet, dass** das Verpackungsmaterial Zellstoff, Kunststoff und/oder Verpackungsmaterial auf Glasbasis und/oder anderes allgemein verwendetes Verpackungsmaterial und/oder ein Verbundmaterial daraus sein kann.

10. Verfahren, bei dem die Verpackung nicht beschädigt wird, um die Verpackung zu orten, indem eine Anzeigeeinrichtung an dem Verpackungsmaterial angebracht wird, **dadurch gekennzeichnet, dass**
eine Anzeigeeinrichtung unter Verwendung des Auftropfverfahrens direkt an der Oberfläche des Verpackungsmaterials in Verbindung mit dem Verpacken vor Abdichten der Verpackung angebracht wird; und
die Verpackung mittels der Anzeigeeinrichtung geortet wird.

11. Verfahren nach Anspruch 10, **dadurch gekennzeichnet, dass** die Anzeigeeinrichtung unter Verwendung des Tintenstrahlverfahrens an der Oberfläche des Verpackungsmaterials angebracht wird.

12. Verfahren nach Anspruch 10 oder 11, **dadurch gekennzeichnet, dass** eine Kennung, die die Verpackung individualisiert, aus der Anzeigeeinrichtung gebildet wird.

13. Verfahren nach Anspruch 12, **dadurch gekennzeichnet, dass** die Kennung eine ein-, zwei- oder dreidimensionale Kennung, eine zusammengesetzte Strich-Kennung, bei der eine lineare, zwei- oder dreidimensionale Strich-Kennung kombiniert worden sind, um ein Symbol auszubilden, oder eine HTML-Strich-Kennung ist.

14. Verfahren nach Anspruch 12 oder 13, **dadurch gekennzeichnet, dass** die Kennung die Chargennummer und die Herstellungszeit des Erzeugnisses umfasst.

15. Verfahren nach einem der Ansprüche 10-14, **dadurch gekennzeichnet, dass**
die durch die an der Verpackung angebrachte Anzeigeeinrichtung gebildete Kennung gelesen wird,
mittels der gelesenen Kennung die Herstellungsdaten der Verpackung aus der Datenbank abgerufen werden,
die in der gleichen Charge enthaltenen Verpackungen geortet werden.

16. Verfahren nach Anspruch 15, **dadurch gekennzeichnet, dass** eine Sortierentscheidung bezüglich der die in der gleichen Charge enthaltenen georteten Verpackungen getroffen wird.

17. Verfahren nach Anspruch 15 oder 16, **dadurch gekennzeichnet, dass** die Kennung mittels automatischer Leseverfahren gelesen wird.

18. Verfahren nach einem der Ansprüche 15-17, **dadurch gekennzeichnet, dass** die in der Kennung enthaltenen gelesenen Informationen durch Verbinden des Erfassungskopfes mit einem Computer und/oder mittels drahtloser Datenübertragungsverfahren übertragen werden.

19. Verpackung zum Mitteilen einer durch eine Undichtigkeit verursachten Veränderung, des Sauerstoffgehaltes oder einer Verschlechterung eines Erzeugnisses in einer Verpackung unter Verwendung einer Anzeigeeinrichtung, die daran angebracht ist, wobei die Verpackung aus Zellstoff, aus Kunststoff und/oder Verpackungsmaterial auf Glasbasis und/oder jedem anderen allgemein verwendeten Verpackungsmaterial und/oder einem Verbundmaterial daraus besteht, **dadurch gekennzeichnet, dass** die Verpackung ausgebildet worden ist, indem die Anzeigeeinrichtung unter Verwendung des Auftropfverfahrens an der Oberfläche des Verpackungsmaterials in Verbindung mit dem Verpacken vor Abdichten der Verpackung angebracht worden ist.

20. Verpackung nach Anspruch 19, **dadurch gekennzeichnet, dass** die Anzeigeeinrichtung unter Verwendung des Tintenstrahlverfahrens an der Oberfläche des Verpackungsmaterials angebracht worden ist.

21. Verpackung nach Anspruch 19 oder 20, **dadurch gekennzeichnet, dass** die Anzeigeeinrichtung ausgebildet worden ist, um eine Kennung auszubilden, die die Verpackung individualisiert.

22. Verpackung nach einem der Ansprüche 19-21, **dadurch gekennzeichnet, dass** die Verpackung eine Lebensmittel-, Arzneimittel- oder Kosmetikverpackung ist.

23. System zum Orten einer Verpackung, **dadurch gekennzeichnet, dass** das System umfasst:
eine Auftropfvorrichtung zum Anbringen der Anzeigeeinrichtung der Verpackung in Verbindung mit dem Verpacken,
eine Lese- und/oder Identifizierungsvorrichtung zum Lesen und/oder Identifizieren der an der Verpackung angebrachten Anzeigeeinrichtung,
eine Datenbank zum Orten der Verpackungen, die in der gleichen Charge mit der Verpackung enthalten sind.

24. System nach Anspruch 23, **dadurch gekennzeichnet, dass** die Lese- und/oder Identifizierungsvorrichtung einen Laser-Scanner, einen CCD-Scanner und/oder Erfassungsköpfe mit Punkt-, Zeilen- oder Matrixstruktur, vorzugsweise CCD- oder CMOS-Zellen, umfasst.

25. System nach Anspruch 23, **dadurch gekennzeichnet, dass** die Lese- und/oder Identifizierungsvorrichtung einen Laser-Scanner, einen CCD-Scanner und/oder eine Vorrichtung umfasst, die Erfassungsköpfe mit Punkt-, Zeilen- oder Matrixstruktur, vorzugsweise CCD- oder CMOS-Zellen, verwendet.

## Revendications

1. Procédé n'endommageant pas l'emballage pour signaler un changement provoqué par une fuite, la teneur en oxygène et/ou la détérioration d'un produit dans un emballage, en attachant un indicateur au matériau d'emballage, **caractérisé par** :
- l'attache d'un indicateur en utilisant la technique d'égouttage directement à la surface du matériau d'emballage conjointement à l'emballage avant de fermer hermétiquement l'emballage ; et
- l'indication, grâce à la décoloration de l'indicateur de fuite de l'emballage, d'un changement de la teneur en oxygène et/ou d'une détérioration du produit.

2. Procédé selon la revendication 1, **caractérisé en ce que** l'indicateur est attaché à la surface du matériau d'emballage en utilisant la technique de jet d'encre.

3. Procédé selon la revendication 1 ou 2, **caractérisé en ce que** l'indicateur est opérationnel lors de l'impression ou est amené en état de fonctionner après l'impression.

4. Procédé selon l'une quelconque des revendications 1 à 3, **caractérisé en ce que** les composants de l'indicateur sont mélangés avant d'attacher l'indicateur à la surface du matériau d'emballage, ou les composants de l'indicateur sont attachés à la surface du matériau d'emballage étape par étape.

5. Procédé selon l'une quelconque des revendications 1 à 4, **caractérisé en ce que** l'indicateur est activé en le soumettant à des conditions qui provoquent et/ou accélèrent l'activation.

6. Procédé selon l'une quelconque des revendications 1 à 5, **caractérisé en ce que** l'indicateur comprend un mélange d'agents de coloration, qui réagit à un changement de la teneur en oxygène, à un changement de pH dans l'emballage, et/ou à un composé produit conjointement à la détérioration du produit.

7. Procédé selon l'une quelconque des revendications 1 à 6, **caractérisé en ce que** la couleur de l'indicateur change suite à l'oxydation et/ou par le biais d'un mécanisme indirect, lequel mécanisme indirect est basé sur le changement de pH et/ou sur une réaction provoquée par un catalyseur, de préférence une enzyme.

8. Procédé selon l'une quelconque des revendications 1 à 7, **caractérisé en ce que** la décoloration de l'indicateur est signalée visuellement et/ou est lue au moyen de techniques de lecture automatique.

9. Procédé selon l'une quelconque des revendications 1 à 8, **caractérisé en ce que** le matériau d'emballage peut être un matériau d'emballage à base de pâte, plastique et/ou de verre, et/ou tout autre matériau d'emballage utilisé généralement et/ou un matériau composé de ceux-ci.

10. Procédé n'endommageant pas l'emballage pour localiser l'emballage en attachant un indicateur au matériau d'emballage, **caractérisé par** :
- l'attache d'un indicateur en utilisant la technique d'égouttage directement à la surface du matériau d'emballage, conjointement à l'emballage avant de fermer hermétiquement l'emballage ; et
- la localisation de l'emballage au moyen de l'indicateur.

11. Procédé selon la revendication 10, **caractérisé en ce que** l'indicateur est attaché à la surface du matériau d'emballage, en utilisant la technique de jet d'encre.

12. Procédé selon la revendication 10 ou 11, **caractérisé en ce qu'**un identifiant individualisant l'emballage est formé à partir de l'indicateur.

13. Procédé selon la revendication 12, **caractérisé en ce que** l'identifiant est un identifiant mono-, bi-, ou tridimensionnel ; un identifiant à barre composé dans lequel un identifiant à barre linéaire, bi- ou tridimensionnel est combiné pour former un symbole ; ou un identifiant à barre HTML.

14. Procédé selon la revendication 12 ou 13, **caractérisé en ce que** l'identifiant comprend le numéro du lot et l'heure de fabrication du produit.

15. Procédé selon l'une quelconque des revendications 10 à 14, **caractérisé par** :
- la lecture de l'identifiant formé par l'indicateur attaché à l'emballage,
- la récupération au moyen de l'identifiant de lecture des données de production de l'emballage à partir de la base de données,
- la localisation des emballages inclus dans le même lot.

16. Procédé selon la revendication 15, **caractérisé en ce qu'**une décision de tri est effectuée en ce qui concerne les emballages localisés inclus dans le même lot.

17. Procédé selon la revendication 15 ou 16, **caractérisé en ce que** l'identifiant est lu au moyen de techniques de lecture automatique.

18. Procédé selon l'une quelconque des revendications 15 à 17, **caractérisé en ce que** les informations lues incluses dans l'identifiant sont émises en connectant la tête de détection à un ordinateur et/ou au moyen de technologies de transfert de données sans fil.

19. Emballage permettant de signaler un changement provoqué par une fuite, la teneur en oxygène, ou une détérioration d'un produit dans un emballage, en utilisant un indicateur attaché à celui-ci, lequel emballage consiste en un matériau d'emballage à base de pâte, plastique et/ou de verre, et/ou tout autre matériau d'emballage utilisé généralement et/ou un matériau composé de ceux-ci, **caractérisé en ce que** l'emballage est formé en attachant l'indicateur, en utilisant la technique d'égouttage à la surface du matériau d'emballage conjointement à l'emballage, avant de fermer hermétiquement l'emballage.

20. Emballage selon la revendication 19, **caractérisé en ce que** l'indicateur est attaché à la surface du matériau d'emballage en utilisant la technique de jet d'encre.

21. Emballage selon la revendication 19 ou 20, **caractérisé en ce que** l'indicateur est formé pour former un identifiant qui individualise l'emballage.

22. Emballage selon l'une quelconque des revendications 19 à 21, **caractérisé en ce que** l'emballage est un emballage pour produits alimentaires, pharmaceutiques ou cosmétiques.

23. Système permettant de localiser un emballage, **caractérisé en ce que** le système comprend :
- un dispositif d'égouttage permettant d'attacher l'indicateur à un emballage conjointement à l'emballage,
- un dispositif de lecture et/ou d'identification permettant de lire et/ou d'identifier l'indicateur attaché à l'emballage,
- une base de données permettant de localiser les emballages inclus dans le même lot que l'emballage.

24. Système selon la revendication 23, **caractérisé en ce que** le dispositif de lecture et/ou d'identification comprend un lecteurlaser, un lecteur CCD et/ou des têtes de détection à structure en point, ligne ou matrice, de préférence des cellules CCD ou CMOS.

25. Système selon la revendication 23, **caractérisé en ce que** le dispositif de lecture et/ou d'identification comprend un lecteur laser, un lecteur CCD et/ou un dispositif utilisant des têtes de détection à structure en point, ligne ou matrice, de préférence des cellules CCD ou CMOS.
